# EUROPEAN PATENT APPLICATION

(11) **EP 0 621 341 A2**
(43) Date of publication of application: **26.10.1994**
(21) Application number: 94106248.1
(22) Date of filing: 21.04.1994
(51) Int. Cl.: C12P 21/08, A61K 39/395, G01N 33/577

(54) **Monoclonal antibodies to bovine cytokines**

(30) Priority: 22.04.1993 US 51489; 15.03.1994 US 213916
(71) Applicant: AMERICAN CYANAMID COMPANY, Wayne New Jersey 07470 (US)
(72) Inventor: Tao, Weng, Belle Mead, New Jersey (US); Dougherty, Ruth M., New Egypt, Jersey 08533 (US); Daley, Michael, Yardley, Pennsylvania 19067 (US)
(74) Representative: Wächtershäuser, Günter, Prof. Dr.

(57) **Abstract**

The present invention relates to monoclonal antibodies which bind to bovine cytokines.

## Description

### Background of the Invention

Cytokines are a diverse group of compounds which exert various effects on the body's immune and inflammatory responses, both by way of direct action on the relevant cells, and by way of interaction and/or regulation of each other. In fact, endogenous cytokines may have both beneficial and adverse effects in the body. For example, the cytokine interleukin (IL)-1 induces lymphocyte proliferation and other related activities which are essential in the body's mounting an immune response to a pathogen. In this regard, it is now known to be useful as an adjuvant in vaccine formulations and in treatment of infectious disease. IL-1 also functions as a mediator of acute inflammation. Although these reactions are desirable in the proper context, and in fact are essential to survival, excessive production of IL-1 can lead to the development of disease. In particular, excess IL-1 can lead to tissue trauma and organ dysfunction. The cytokine TNFα also has activities very similar to those of IL-1, but was originally identified by its association with necrosis of certain tumor types, and has been suggested as being the primary agent responsible for inducing shock and related syndromes.

IL-2 is a cytokine, the major function of which is to stimulate lymphocyte proliferation and maturation, an obviously important function in immune response. Clinically, it can be used in treatment of tumors, and also, like IL-1, as a vaccine adjuvant and in treatment of infectious diseases. However, excess IL-2 can also produce such adverse effects as pulmonary and hepatic lymphocyte infiltration, lymphoid pleural effusions, eosinophilia, thrombocytopenia, and capillary leakage.

Another hematopoietic factor, granulocyte macrophage colony stimulating factor (GM-CSF), is also involved in the immune and inflammatory response processes. IL-1 is actually responsible for stimulating production of GM-CSF from endothelial cells and other connective tissue cells. GM-CSF itself causes production of new macrophages and neutrophils and primes mature macrophages and neutrophils. Therapeutically, GM-CSF has been used in treatment of cytotoxic injury, as well as in bone marrow manipulation.

Both the natural and therapeutic functions of these cytokines are applicable in both man and animals. In particular, cytokine therapy is useful in food animals such as cows, and the roles of cytokines in the inflammatory and immune responses in cows has also been well established. The ability to monitor and/or alter cytokine levels in such animals can provide both therapeutic and prophylactic benefits. In addition, because of their use as food animals, for regulatory purposes, it may be essential to be able to quantify residual levels of therapeutically administered cytokines in serum or milk. Until now, the reagents necessary to achieve this have not been available. The present invention now provides reagents useful in making such determinations.

### Summary of the Invention

The present invention relates to monoclonal antibodies which react with cytokines, particularly bovine cytokines such as interleukins, GM-CSF and TNF. In a preferred embodiment, the antibody reacts with IL-1 (α or β), IL-2 or GM-CSF. In certain embodiments, it is preferred that the antibody be capable of neutralizing the target cytokine.

The invention also provides novel diagnostic methods. For example, the antibody can be used in an ELISA or other rapid detection systems to identify the presence of the cytokine in a sample. Thus, a method is provided for determination of the level of a given cytokine in tissue, or body fluids, such as plasmas, serum or milk. In one embodiment, the level of cytokine is indicative of the stage of development of a disease, prior to the development of symptoms. In another embodiment, the method provides a reliable indicator of the residual cytokine remaining in serum or milk of an animal which has been treated therapeutically with a cytokine.

The antibodies, per se, can also be used directly or indirectly as therapeutic agents. The antibody can be used in formulation with the corresponding cytokine to prolong the half-life of the cytokine and to enhance the intended response to the cytokine. The antibody alone can be administered in a therapeutic method for treatment of pathological conditions, e.g., conditions involving inflammation, so as to neutralize the cytokine which mediates the pathological condition.

### Detailed Description of the Invention

The monoclonal antibodies of the present invention are prepared by immunizing an appropriate host animal, such as a mouse, rat, goat, sheep or rabbit, with a substantially pure sample of the cytokine of interest, such as a bovine cytokine, or more preferably, a recombinantly produced bovine cytokine. Methods for production of recombinant bovine cytokine are known and are described in, for example, U.S. 5,106,733; 4,894,333; 4,879,374; 4,882,282; and 5,006,465. Spleen cells are collected from immunized animals, and fused with an appropriate immortal cell line, such as SP2/O, X63Ag8. Selection for hybridomas is carried out in HAT medium. Specificity of the antibodies produced by hybridomas is tested in an ELISA with the purified cytokine of choice as the substrate. Cells producing monoclonal antibodies of the desired specificity are injected into pristine-primed histocompatible hosts and ascites fluid collected. Monoclonal antibodies are purified by techniques standard in the art.

A number of monoclonal antibodies which react with bovine cytokines are obtained in this manner. Utilizing IL-1β as immunogen, several hybridomas are obtained. A preferred antibody for this group is MoAb 8.1, produced by HB11244. MoAb 8.1 is characterized as having γ₁ heavy chains and κ light chains. Using IL-2 as immunogen, several hybridomas are also produced; a preferred antibody from this group is 14.1, produced by HB11243. This antibody is characterized as having γ₁ heavy chains and κ light chains. Several anti-GM-CSF antibodies are also produced, of which the preferred antibody is 6.1, produced by hybridoma Hb11245. This antibody is characterized as having γ₁ heavy chains and κ light chains.

Additional monoclonal antibodies can be made as described above or in the following examples, utilizing purified cytokines as immunogens. In addition to IL-1, IL-2 and GM-CSF, purified cytokines such as IL-3, IL-4, IL-5, IL-6, IL-7, IL-8, IL-9, IL-10, IL-11, IL-12, IL-13, M-CSF, TGFβ or TNF can also be used, and methods for making same are also well documented in the art.

The availability of such antibodies enables a number of diagnostic as well as therapeutic methods. In connection with the therapeutic utility of the cytokines per se, the monoclonal antibodies can be used in standard ELISAs to monitor the presence of residual cytokines in serum or milk, which can be important in meeting regulatory requirements. Such ELISAs can be conducted using monoclonals alone, or a combination of monoclonal and polyclonal antibodies. For example, briefly, a capture antibody is bound to a solid substrate such as a multiwell plate, and contacted with the sample obtained from a treated animal to be tested. After washing to remove any unbound material, a detectably labelled second antibody (polyclonal or monoclonal) is added to the plate; if binding has occurred, the presence of the detectable label will be visible, thereby indicating the presence of cytokine in the sample. Those skilled in the art will also recognize that the antibody can also be used in standard radioimmunoassays (RIAs), or with other comparable detectable labelling methods.

Such ELISAs or RIAs can also be used as diagnostic tools to monitor cytokine levels in animal bodily fluids or tissue. As noted above, certain cytokines, in particular IL-1 and TNF, are actively involved in the inflammatory response, and particularly in toxic shock. The monoclonal antibodies can be used to determine baseline levels of cytokines in various bovine tissues and fluids. Then, the documentation of normal or basal levels of cytokines can be used as an indicator to determine when above normal levels of cytokines are present in bodily fluids. High or rising levels of these cytokines in animal sera can be indicative of acute or chronic infection before any actual symptoms appear. Thus, such diagnostic tools provide a means for monitoring the health of a herd, by routine periodic checks on these cytokine levels by the methods described above.

The antibodies of the present invention can also be used therapeutically. For example, in the cases in which the cytokines are involved in the inflammatory response, it may be desirable to reduce endogenous IL-1 or TNF levels in order to avoid the pathological sequelae which typically result from high IL-1 and/or TNF levels. To this end, administration of a monoclonal antibody which not only binds but also neutralizes the biological activity of the cytokine can prevent the downstream adverse effects of the cytokine. For example, anti-IL-2 antibody 14.1 noted above is capable of neutralizing IL-2 activity, presumably by binding with an epitope which is also responsible for binding with the IL-2 receptor on target cells. Neutralizing antibodies can be identified in standard bioassays specific for the cytokine of interest. A neutralizing antibody for a given cytokine can be used to inhibit the biological response induced by that cytokine.

Also, non-neutralizing anticytokine monoclonal antibodies can be used therapeutically to enhance the activity of either endogenous or exogenously administered cytokines. Specifically by binding with its target cytokine in vivo, the antibody may delay the cytokine's clearance from circulation, thereby prolonging the cytokines half-life and increasing the probability it will interact with the appropriate target cells. This may be of particular interest in connection with the use of cytokines such as IL-2 and GM-CSF in enhancement of desirable immune response.

Such antibodies are also useful in purifying endogenous cytokines, identifying receptor binding epitopes in the cytokine and studying species specificity of cytokines.

The invention is further explained with reference to the following non-limiting examples.

### EXAMPLES

### EXAMPLE 1 Antibody Preparation

### A. Production of Monoclonal Antibodies

### 1) Hybridoma Construction

Balb C mice are immunized with 25 µg purified r-BoIL-1, r-BoIL-12 or r-BoGM-CSF (Immunex Corp, Seattle, WA) per injection. Primary injections are given subcutaneously in complete Freund's adjuvant. Five subsequent injections of 25 µg are given intraperitoneally in PBS. Mice are boosted three days prior to fusion date. Sensitized spleens from two mice are fused with SP2/O mouse myeloma cells. Selection for hybridomas are carried out in HAT media. Antibody producing cells are identified by ELISA screening of supernatants. Cells with desired specificities are cloned by limited dilution over a thymocyte feeder layer. Antibody isotopes are determined by ELISA. Several hybridomas are identified which produce either anti-IL-1 antibodies, anti-IL-2 antibodies or anti-GM-CSF antibodies, depending on the immunogen.

### 2) Preparation of Ascites

Pristane-primed Balb C mice are injected intraperitoneally with 0.2 ml monoclonal antibody producing cells at 2 x 10⁷ cells per ml. Cells are suspended in PBS; injection and ascites preparation are achieved in accordance with standard protocols, such as are described in Immuno-chemistry in Practice (Alan Johnstone/Robin Thorpe).

### B. Production of Polyclonal Antibodies

Rabbits (New Zealand White) are immunized with 50 µg of antigen (r-BoIL-1, r-BoIL-2 or r-BoGM-CSF) four times at two-week intervals. The first injection is carried out in Freund's complete adjuvant. Second injections are carried out in Freund's incomplete adjuvant. Subsequent injections are in PBS. The blood is collected two weeks after each immunization. The blood is allowed to clot at room temperature and serum is harvested after brief centrifugation.

### C. Purification of Antibodies

Both monoclonal ascites and polyclonal serum are purified with MAB Trap G column (Pharmacia, LKB). Recommended manufacturer's guidelines for the procedure are followed. Briefly, prior to collection of purified antibody fractions, 60 µl of neutralizing buffer is added to collection tubes per ml of fraction. 5 ml of ascites or serum is centrifuged at 10,000 g for 10 minutes and passed through a .22 µm filter. The sample is diluted 1:1 with binding buffer. Binding buffer is then added to the Protein G Sepharose 4 FF column and allowed to drain. The prepared sample is then added to the column first and allowed to absorb into the gel. Binding buffer is then passed through the column to elute the unbound materials. The bound IgG is eluted by filling the column with elution buffer. The antibody is then collected in the prepared collection tubes.

### D. Protein Concentration Determination

Protein assays are performed on aliquots of purified monoclonal antibody samples using the BCA reagent (Pierce) and recommended manufacturer's guidelines for the procedure. The optic density of the samples are measured using a spectrophotometer (Pharmacia LKB) at 562 mm wavelength.

### II. Neutralization Activity

### A. IL-1 Neutralization

The ability of an antibody to neutralize IL-1 is determined by the thymocyte costimulation assay. All assays are performed in 96-well tissue culture plates.

### 1) Preparation of Effector Cells

Thymuses are aseptically removed from C3h mice or Dunkin Hartley guinea pigs. Cells are minced between two sterile glass slides, washed in RPMI with or without serum (RPMI without serum is used in the polyclonal antibody thymocyte assay), and resuspended in media.

### 2) PHA Dose Responsive Curve

A dose responsive curve of PHA is established for mouse thymocytes. Briefly, two-fold dilutions of PHA are carried out in RPMI to give the final concentration range from 0.001 µg/ml to 100 µg/ml. Thymocytes are seeded at 7.5 x 10⁶ cells per well. The plates are incubated for 66 hours at 37°C in an atmosphere of 5% CO₂. The cells are then pulsed with 50 µg/well of [methyl-3H]-thymidine at a concentration of 10 µCi/ml. The plates are incubated an additional 6 hours at 37°C, 5% CO₂. Following the incubation, cell cultures are harvested onto a filter paper using a TOMTEC cell Harvester and the radionucleotide incorporation measured on a Beta plate reader (Pharmacia, LKB). The suboptimal dose of PHA (2.5 µg/ml) is chosen for the following thymocytes costimulator assays.

### 3) IL-1 Dose Responsive Curve

Bovine IL-1 is serially diluted in assay media containing PHA (2.5 µg/ml). The final concentrations of IL-1 are from 0.0001 ng/ml to 10 ng/ml. Mouse thymocytes are added at 7.5 x 10⁶ cell per well. The reactions are carried out as described above.

### 4) Inhibition of IL-1 Activity by anti-r-BoIL-1 Antibody

### a) Rabbit Polyclonal Antibody

Suboptimal concentrations of IL-1 and PHA (determined by the thymocyte costimulator assay where there is 50-80% of maximum cell proliferation) are chosen for the following experiments. r-BoIL-1 is incubated with different dilutions of purified rabbit polyclonal anti-r-BoIL-1 antibody at 37°C for 3 hours. The assay media contains no serum in order to avoid any effect that serum may impose. Purified rabbit polyclonal anti-r-BoIL-2 antibody is included as a control. Following incubation of IL-1 with anti-r-BoIL-1 body, equal volume of thymocytes in 20% serum are added at 7.5 x 10⁵ cell per well. The reactions are carried out as described.

### b) Mouse Monoclonal Antibodies

Three fold serial dilutions of r-BoIL-1 are carried out in assay medium, the final assay concentrations ranging from 100 ng/ml to 0 ng/ml for the standard. Four experimental concentrations of r-BoIl-1 ranging from 100 ng/ml to 0.134 ng/ml are used to evaluate the neutralizing ability of purified anti-IL-1 antibodies. An equal volume of PHA diluted in assay medium is then added to the IL-1 dilution series. The final assay concentration of PHA is 2 µg/ml. Antibodies are serially diluted 1:2 in assay medium and added to the experimental IL-1 dilutions at final concentrations of 1:25, 1:50, 1:100 and 1:200. 100 µl of each sample is then added in triplicate to wells in a 96 well flat bottomed microtiter plate (Corning). The antibody and IL-1 are then allowed to incubate for 30 minutes at 37°C in 5% CO₂ and air.

Freshly isolated thymocytes are suspended to 1.5 x lO⁷ viable cells/ml in assay medium. (The thymocyte preparation is consistently done following the preparation of the assay plates.) 100 µl of the prepared cell suspension is then added to the 100 µl volume already present in the wells, for a final volume of 200 µl/well.

Plates are then incubated for 68 hours at 37°C in an atmosphere of 5% CO₂ in air. Cultures are then pulsed with 50 µl/well of [methyl-3H]-thymidine (NEN, 20.0 Ci/mMol) at a concentration of 10 µCi/ml. The cultures are then incubated for an additional 6 hours at 37°C, 5% CO₂ in air. Following the incubation, cell cultures are harvested onto a filter paper using a TOMTEC cell harvester, and the radionucleotlde incorporation is measured on a Beta plate reader (Pharmacia LKB).

Results of the IL-1 neutralization assay are shown in Table 1, below. The assay shows that the polyclonal sera has good neutralizing activity, while none of the specific monoclonals tested were effective in neutralizing IL-1.

### B. IL-2 Neutralization

The ability of antibodies to neutralize IL-2 activity is evaluated by the BT₂ assay, as follows:
Two-fold serial dilutions of r-BoIL-2 are done in growth medium, the final assay concentrations ranging from 100 ng/ml to 0 ng/ml for the standard. Four experimental concentrations of r-BoIL-2 ranging from 100 ng/ml to 0.195 ng/ml are used to evaluate the neutralizing ability of purified anti-IL-2 antibodies. Antibodies are serially diluted 1:2 in growth medium and added to the experimental dilutions of IL-2 at final concentrations of 1:25, 1:50, 1:100 and 1:200. 100 µl of each sample is then added in triplicate to wells in a 96 well flat-bottomed microtiter plate (Corning). The antibody + IL-2 is then allowed to incubate for 30 minutes at 37°C in 5% CO₂ and air.

BT₂ cells, after being washed twice, are suspended to 8 x lO⁴ cell/ml after in growth medium. The BT₂ cell preparation is consistently done following the preparation of the plates. 100 µl of the prepared cell suspension is then added to the 100 µl present in the assay wells for a final volume of 200 µl/well. Plates are then incubated for 48 hours at 37°C in an atmosphere of 5% CO₂ in air. Cultures are then pulsed with 50 µl/well of [methyl-3H]-thymidine (NEN, 20.0 Ci/mMol) at a concentration of 10 µCi/ml. The cultures are then incubated for an additional 4 hours at 37°C, 5% CO₂ in air. Following the incubation, cell cultures are harvested onto a filter paper using a TOMTEC cell harvester, and the radionucleotide incorporation is measured on a Beta plate reader (Pharmacia LKB).

Results of the IL-2 neutralization assays are also shown in Table 1. It is noted that several antibodies, both polyclonal and monoclonal, have neutralizing activity against IL-2. An IL-2 neutralizing antibody can be used to confirm the presence of IL-2 in bodily fluids. Also, it can be used to regulate the level of IL-2 in vivo.

**TABLE 1**

| AB | Isotype (H,L) | Protein (µg/ml) | Neutralizing Activity* |
|---|---|---|---|
| IL-I Mab (M α Bo) | | | |
| 8.1 | γ1,κ | 728.65 | - |
| 9.3 | γ1,κ | 695.42 | - |

| IL-1 Po1y Ab (R α Bo) | | | |
|---|---|---|---|
| "Steve" | | 2800 | + |

| IL-2 Mab (R α Bo) | | | |
|---|---|---|---|
| 12.8 | γ1,κ | 414.31 | +- |
| 13.1 | γ1,κ | 710.69 | +- |
| 14.1 | γ1,κ | 676.56 | + |
| 15.7 | γ1,κ | 105.34 | +- |
| 19.1 | γ1,κ | 627.15 | - |
| 21.1 | γ1,κ | 575.07 | - |
| 22.2 | γ1,κ | 379.27 | +- |
| 24.6 | γ1,κ | 725.05 | + |

| IL-2 Poly Ab (R α Bo) | | | |
|---|---|---|---|
| "Mike" | | 3000 | + |

| GM-CSF-Mab (MoaB) | | | |
|---|---|---|---|
| 6.1 | γ1,κ | 380 | - |
| 10.1 | γ1,κ | 320 | - |
| 11.1 | γ1,κ | 320 | - |
| 17.2 | γ1,κ | 800 | - |
| 20.1 | γ1,κ | 875 | - |

| GM-CSF PolyAB (RαBO) | | | |
|---|---|---|---|
| "Bill" | | 2800 | - |

| | | | |
|---|---|---|---|
| * The neutralizing activity for IL-1 is examined by thymocyte costimulator assay; neutralizing activity for IL-2 is examined by IL-2 dependent BT₂ cell proliferation assay; and neutralizing activity for GM-CSF is examined by bone marrow cell proliferation assay. | | | |

### C. GM-CSF Neutralization

The ability of antibodies to neutralize GM-CSF activity is evaluated by the bone marrow assay.

### 1. Reagents and Media

Dilution media contains RPMI 1640; 10% FCS; 200 units/ml penicillin; 200 µg/ml streptomycin; 0.2 Mm gentamycin; 2 Mm glutamine; and 10 units/ml heparin. Assay media contains Eagles MEM; 10% FCS; 5% Horse Serum; 100 units/ml penicillin; 100 µg/ml streptomycin; and 2 Mm glutamine. 60% isotonic percoll comprises 40% D-PBS to 60% isotonic percoll (1 part 10x D-PBS to 9 parts stock percoll).

### 2. Preparation of Bone Marrow Cells

Femurs are collected from 1-5 day old calf, all muscle scraped off bone and washed with 70% ethanol. Using sterile instruments, the tips are cut off of the bone as close to the top of the shaft as possible; the younger the calf, the closer to the epiphysis is the marrow. Once the tip of the marrow is found in a clean room or hood, a spoon tipped sterile spatula is used to scrape the marrow out of the bone shaft into petri plates containing dilution media. The marrow is diluted approximately 4x with this process. The marrow is passed through a cell dissociation sieve (Sigma CD-1) with a 60 mesh screen. The marrow is diluted at least 3x during this process.

Plates are incubated 45 minutes at room temperature to allow adherence, then non-adherent cells are collected and layered over 60% percoll. This mixture is centrifuged 30 minutes, 500 x g at room temperature. Cells at the interface are collected (above rbc's and below fat and "fuzzy" layer). Cells are centrifuged 10 minutes, 300 x g room temperature, then washed with dilution media and spun 10 minutes, 300 x g at room temperature.

### 3. Neutralizing Activity of Anti-GM-CSF Antibody

Cells are resuspended in assay media, counted, and diluted to 1.25 x 10⁶ cells/ml. 100 µg/well of cells and 100 µl of r-boGM-CSF are added with or without antibody in a 1:3 serial dilution starting at 500 ng/ml, and incubated at 30°C, 5% CO₂, 95% humidity for 72 hours. They are then pulsed with 2 µCi/well [³H]-thymidine for 18 hours and frozen prior to harvesting and counting.

### III. DIAGNOSTIC ELISAS

### A. Direct Binding ELISA

A plate is coated overnight at room temperature with cytokine, at 0.5 micrograms per milliliter in PBS, 100 microliters per well. The plate is then washed 4x with 0.05% Tween 20/PBS. The plate is blocked with 1% BSA/PBS, 200 µl/well, and incubated at 37°C for 1 hour. The contents are then dumped. Tissue culture supernatants or antibodies are added at desired concentration and serially diluted accordingly in PBS/BSA. Final volume in wells should be 100 microliters. At least one well is left with no antibody so that background can be determined, i.e., only PBS/BSA. Samples are incubated 2 hours at 37°C, then washed 4x with PBS/Tween 20. An appropriate purified secondary antibody is added at 1:500 or 1:1000 (according to package insert) that is conjugated to alkaline phosphatase-100 µl/well, diluted in PBS/BSA, and incubated 1 hour at 37°C. The samples are washed 4x with PBS/Tween 20. PNPP (p-nitrophenyl phosphate) is prepared according to manufacturers' directions, substrate is added at 100 µl/well. PNPP is prepared according to package insert, 100 µl per well. Each well is read at 405 nm at 15, 30 and 60 minutes.

### B. Capture ELISA for Cytokines

Plates are coated overnight at room temperature with the appropriate purified antibody or antibodies. The antibody may be a single monoclonal antibody to the cytokine of multiple antibodies to different epitopes on the cytokine, or polyclonal antibodies to cytokine. The antibody/antibodies are diluted in PBS, to a concentration of 100 µl/well. The wells are washed four times with 0.05% Tween 20/PBS, blocked with 1% BSA/PBS at 200 µl/well and incubated for 1 hour at 37°C. The liquid contents are then removed. In tests for developing a standard curve for a specific cytokine, in a 96-well plate, 100 µl of diluent, such as 1% BSA/PBS, or milk or plasma, is added to all wells but one. Serial dilutions are made from 50 µl into 100 µl across wells 1-11, with the twelfth well containing no cytokine. For unknowns, 100 µl of the samples are added to the wells; the standard curve wells should contain the sane diluent as the unknown samples.

The prepared wells are incubated for 2 hours at 37°C, and then washed four times with PBS/Tween 20. The purified secondary antibody is then added. This secondary antibody may be monoclonal or polyclonal, depending on the nature of the first antibody, and may be labelled or unlabelled. The mixture is incubated one hour at 37°C and washed four times in PBS/Tween 20. If the secondary antibody is unlabelled, at this stage, a tertiary labelled anti-Ig antibody is then added, diluted in PBS/BSA, at 100 µl per well. Whether the labelled antibody is the secondary or tertiary antibody, the mixture is incubated 30-60 minutes at 37°C, and washed with PBS/Tween 20. The appropriate substrate for the labelled conjugate is then added, in accordance with manufacturer's directions, at 100 µl/well. Readings are taken at 405 nm after 30-60 minutes.

### DEPOSIT OF BIOLOGICAL MATERIALS

The following biological materials have been deposited with the American Type Culture Collection, 12301 Parklawn Drive, Rockville, Maryland, under the Budapest Treaty, and have the indicated Accession Numbers:

| Description | Accession No. |
|---|---|
| Hybridoma 14.1 (anti-rBoIL-2) | ATCC HB11243 |
| Hybridoma 8.1 (anti-rBoIL-1β) | ATCC HB11244 |
| Hybridoma 6.1 (anti-rBoIL-GM-CSF) (anti-rBoGMLSF) | ATCC HB11245 |

## Claims

1. A monoclonal antibody which binds with a bovine cytokine.

2. The antibody of Claim 1 which binds with a cytokine selected from the group consisting of IL-1, IL-2, IL-3, IL-4, IL-5, IL-6, IL-7, IL-8, IL-9, IL-10, IL-11, IL-12, IL-13, GM-CSF, TNF, TGFβ and G-CSF.

3. The antibody of Claim 2 which binds IL-I.

4. The antibody of Claim 3 which neutralizes IL-1 activity.

5. The antibody of Claim 3 which competitively inhibits binding of the antibody produced by the hybridoma deposited as ATCC 11244 to IL-1.

6. The antibody of Claim 3 which is produced by the hybridoma deposited as ATCC 11244.

7. The antibody of Claim 2 which binds IL-2.

8. The antibody of Claim 7 which neutralizes IL-2 activity.

9. The antibody of Claim 7 which competitively inhibits binding of the antibody produced by the hybridoma deposited as ATCC 11243 to IL-2.

10. The antibody of Claim 7 which is produced by the hybridoma deposited as ATCC 11243.

11. The antibody of Claim 2 which binds with GM-CSF.

12. The antibody of Claim 11 which competitively inhibits binding of the antibody produced by the hybridoma deposited as ATCC HB 11245.

13. The antibody of Claim 11 which is produced by the hybridoma deposited as ATCC HB11245.

14. A pharmaceutical composition comprising an effective amount of an antibody which binds with a bovine cytokine.

15. The composition of Claim 14 in which the antibody binds with a cytokine selected from the group consisting of IL-1, IL-2, IL-3, IL-4, IL-5, IL-6, IL-7, IL-8, IL-9, IL-10, IL-11, IL-12, IL-13, GM-CSF, TNF, TGFβ and G-CSF.

16. A method for detecting the presence of a cytokine in bovine bodily fluid sample comprising contacting the sample with a substrate-bound first antibody which binds the cytokine, permitting time for an antibody-cytokine complex to form; contacting the sample with a second antibody which binds the cytokine, permitting time for a second antibody-cytokine complex to form; and detecting the presence or absence of the first antibody cytokine-second antibody complex, wherein at least one of the antibodies is a monoclonal antibody according to Claim 1.

17. The method of Claim 16 in which the second antibody is detectably labelled.

18. The method of Claim 16 in which the complex is detected by addition of a third, detectably labelled antibody which binds with the second antibody.

19. The method of Claim 16 in which the cytokine is selected from the group consisting of IL-1, IL-2, IL-3, IL-4, IL-5, IL-6, IL-7, IL-8, GM-CSF, TNF and G-CSF.

20. A method for determining the presence of inflammatory or disease conditions in a bovine comprising periodically monitoring levels of IL-1 or TNF in bovine bodily fluid, whereby increasing levels of IL-1 or TNF in a monitoring period is indicative of the presence of an inflammatory or disease condition.

21. A method of potentiating the activity of a cytokine, comprising providing an effective amount of an antibody which binds to the cytokine.

22. The use of an antibody which binds to a cytokine for the manufacture of a medicament for the purpose of altering a host's biological response to the cytokine.

23. The use of an antibody which binds to a cytokine for the manufacture of a medicament for the purpose of potentiating a host's biological response to the cytokine.
